# EUROPEAN PATENT APPLICATION

(11) **EP 2 386 323 A2**
(43) Date of publication of application: **16.11.2011**
(21) Application number: 10729356.5
(22) Date of filing: 12.01.2010
(51) Int. Cl.: A61M 5/158, A61B 17/064

(54) **PLASTIC SURGICAL INJECTION NEEDLE HAVING SURGICAL THREAD FOR WRINKLE REMOVAL**

(30) Priority: 12.01.2009 KR 20090002346
(71) Applicant: Lee, Hee-Young, Jeollabuk-do 573-574 (KR)
(72) Inventor: Lee, Hee-Young, Jeollabuk-do 573-574 (KR)
(74) Representative: Grünecker, Kinkeldey, Stockmair & Schwanhäusser
(86) International application number: PCT/KR2010/000195
(87) International publication number: WO 2010/080014

(57) **Abstract**

Disclosed is an injection needle for plastic surgery prefilled with one or more surgical threads for wrinkle removal. The injection needle for plastic surgery is designed to have a sharp tip through which the surgical threads are inserted into a human body and a hollow cylindrical injection portion accommodating the surgical threads therein. Each of the surgical threads is 0.8 to 10 cm long and has a plurality of projections in different directions. The projections are symmetrically formed with respect to the axis of the thread to gather soft tissues of the skin or to stretch epidermal tissues.

## Description

### [Technical Field]

The present invention relates to an injection needle for plastic surgery through which surgical threads are inserted into the epidermal layer of the skin to remove wrinkles on the skin.

### [Background Art]

Numerous plastic surgical treatments, such as facelift, filler insertion, Botox injection and laser skin resurfacing, have usually been used to remove wrinkles around the face, lower jaw and neck skins. Non-surgical wrinkle removal treatments using surgical threads instead of surgical knives have attracted more and more attention in recent years because they are much simpler to perform than conventional surgical treatments and do not cause substantial inconvenience to the daily life of those treated.

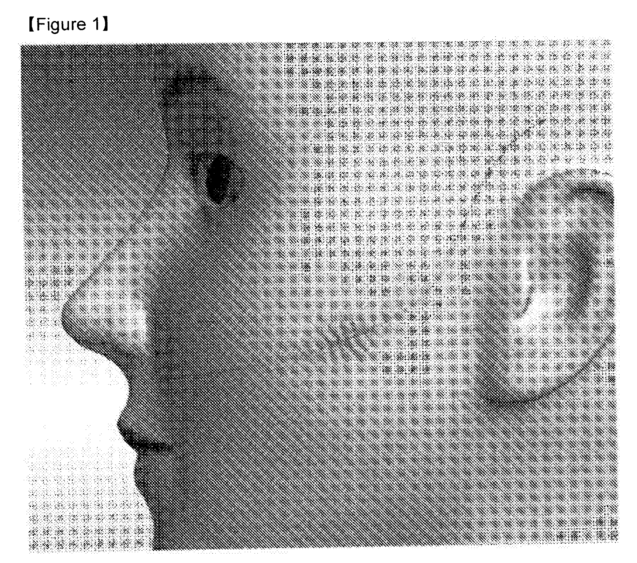

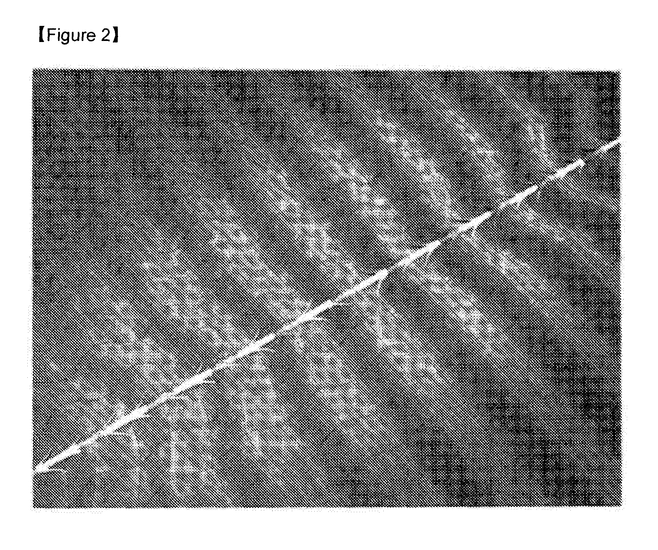

Such a non-surgical wrinkle removal treatment is illustratively shown in FIGS. 1 and 2. This treatment uses a surgical thread having directional projections like thorns. After the surgical thread is inserted into the epidermal layer of the skin, the projections separate the adjacent skin tissues away from each other or prevent them from being spaced apart from each other.

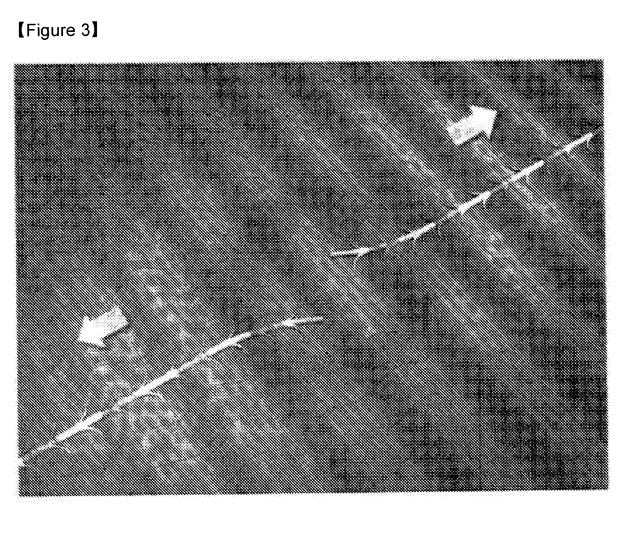

Unlike natural skin, however, the surgical thread is not sufficiently elastic and is stiffly fixed in view of its characteristics. As shown in FIG. 3, the surgical thread may be cut when a treated person smiles or opens his/her mouth wide.

Further, the limited elasticity of the surgical thread restricts the movement of the face toward the axis of the thread and causes inconvenience in expressing various emotions.

Collagen fibers of the skin are micrometer-sized small units and are arranged in various directions. Collagen fibers contain elastin as an elastic component positioned therebetween. Elastin has a certain modulus of elasticity and some extensibility, whereas general surgical threads substantially lack extensibility and lose elasticity over the entire region because of their excessive length. Thus, the surgical threads suffer from the above problems.

In an attempt to solve the above problems, the use of threads made of elastic material such as silicone is also considered. However, since silicone-made projections of the threads are not structurally strong enough to fasten the skin tissues, they are liable to be arranged randomly and are likely to be exposed to the outside. Further, the projections are disadvantageous in service time owing to their poor vulnerability to repeated movements. These disadvantages limit the use of the silicone-made threads.

In another attempt to solve the above problems, the use of relatively short surgical threads is considered to remove skin wrinkles. However, this treatment causes severe bleeding and is complicated and difficult to perform, which make it substantially impossible to use the short surgical threads.

### [Disclosure]

### [Technical Problem]

The present invention has been made in view of the above problems, and it is an object to provide an injection needle for plastic surgery through which short surgical threads can be inserted into the face or neck skin to remove wrinkles around the face or neck skin in an easy and safe manner, so that problems arising from the use of long surgical threads can be solved.

It is another object of the present invention to an injection needle for plastic surgery through which short surgical threads having projections in various shapes can be inserted into the skin.

### [Technical Solution]

According to the present invention, there is provided an injection needle for plastic surgery prefilled with one or more surgical threads for wrinkle removal, designed to have a sharp tip through which the surgical threads are inserted into a human body and a hollow cylindrical injection portion accommodating the surgical threads therein, wherein each of the surgical threads is 0.8 to 10 cm long and has a plurality of projections in different directions that are symmetrically formed with respect to the axis of the thread to gather soft tissues of the skin or to stretch epidermal tissues.

The projections may be arranged helically along the axis of each surgical thread.

The injection portion may have a diameter of 18 to 26 gauges.

Each of the surgical threads may have a diameter of 0.25 to 0.80 mm.

Each of the surgical threads may be made of a monofilament of a biodegradable polymer selected from polydioxanone (PDS), Monocryl, polycaprolactone (PCL), poly(glycolic acid) (PGA), poly(lactic acid) (PLA) and poly(lactic-co-glycolic acid) (PLGA).

Each of the surgical threads may be made of a monofilament of polypropylene or nylon.

Each of the surgical threads may be a shaped yarn.

The projections may be formed at corners of a polygonal cross-section of each surgical thread.

### [Advantageous Effects]

The injection needle of the present invention can insert short surgical threads into the soft tissue of the skin in a stable and easy manner. The short surgical threads inserted into the soft tissue to connect the epidermal tissues therebetween and have elasticity to some extent over the entire region thereof. An external force applied to the inserted short surgical threads is rapidly dispersed into the skin. Since the surgical threads are elastic likewise the soft tissues of the skin, they become highly mobile after treatment and their anti-wrinkling effects can be continued for a long period of time.

In addition, the injection needle of the present invention can insert a plurality of surgical threads into the skin at one time, so that the risk of bleeding can be minimized.

### [Description of Drawings]

FIG. 1 schematically shows a state in which a surgical thread is injected into the skin to remove wrinkles in accordance with the prior art.
FIG. 2 shows a state in which a surgical thread is injected to remove wrinkles in accordance with the prior art.
FIG. 3 is a view showing a problem arising when a surgical thread is injected into the skin to remove wrinkles in accordance with the prior art.
FIG. 4 is a cross-sectional view of an injection needle for plastic surgery prefilled with surgical threads for wrinkle removal according to an embodiment of the present invention.
FIG. 5 illustrates various shapes of projections of surgical threads prefilled in injection needles for plastic surgery according to exemplary embodiments of the present invention.
FIG. 6 illustrates various cross-sections of surgical threads prefilled in injection needles for plastic surgery according to exemplary embodiments of the present invention.
FIG. 7 is a cross-sectional view illustrating a state in which surgical threads for wrinkle removal are prefilled in an injection needle for plastic surgery according to an embodiment of the present invention and a push pin is used to inject the surgical threads into a human body.
FIG. 8 shows a state in which surgical threads are injected using an injection needle according to an embodiment of the present invention.
FIG. 9 shows surgical threads injected using an injection needle according to an embodiment of the present invention to remove wrinkles.
FIG. 10 shows a state in which surgical threads are injected using an injection needle according to another embodiment of the present invention.
FIG. 11 shows surgical threads injected using an injection needle according to another embodiment of the present invention to remove wrinkles.
FIG. 12 schematically shows a state in which surgical threads are injected into the skin to remove wrinkles in accordance with the present invention.

### <Explanation of main reference numerals>

- 100:: Injection needle for plastic surgery 102: Injection portion
- 200:: Surgical thread 202: Projections 300: Push pin

### [Best Mode]

Preferred embodiments of the present invention will now be described in detail with reference to the accompanying drawings. It should be noted that whenever possible, the same reference numerals will be used throughout the drawings and the description to refer to the same or like parts. In describing the present invention, detailed descriptions of related known functions or configurations are omitted in order to avoid making the essential subject of the invention unclear.

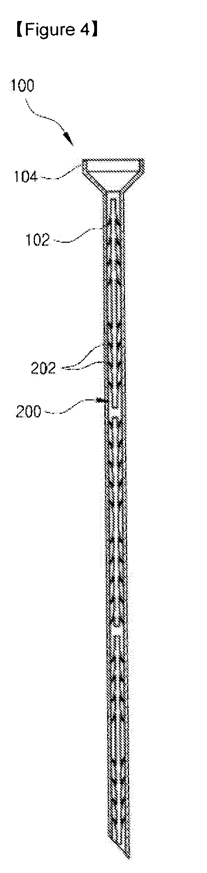

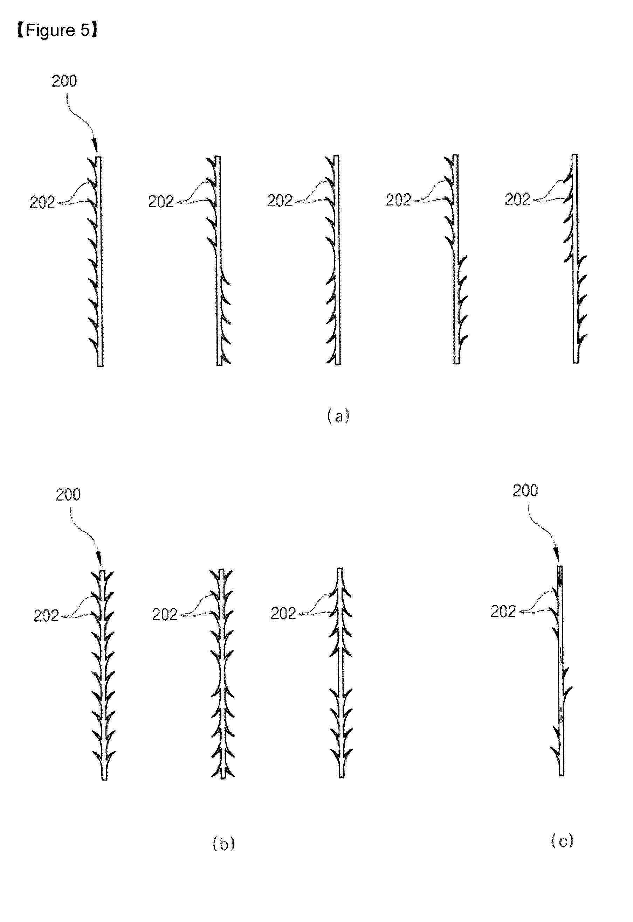

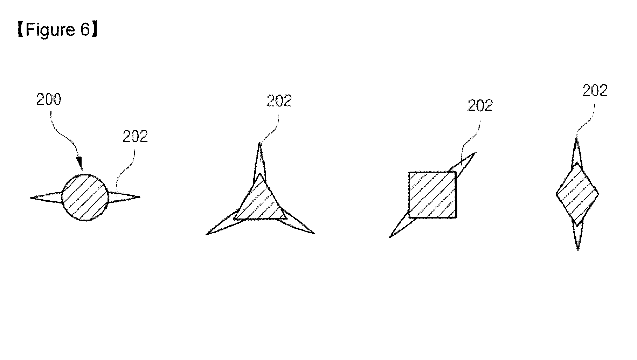

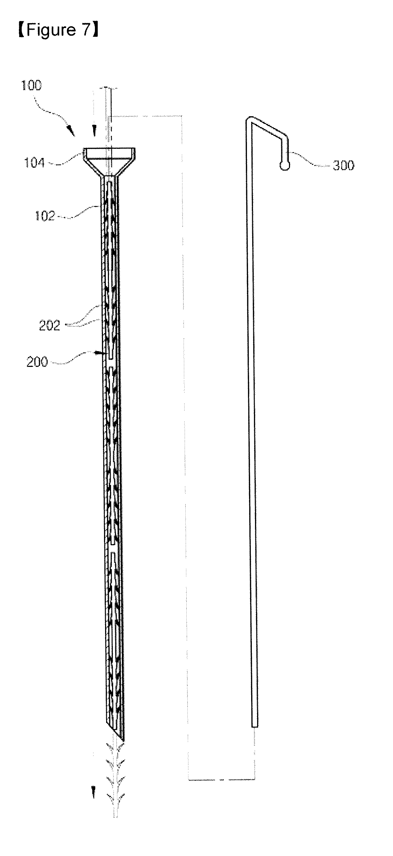

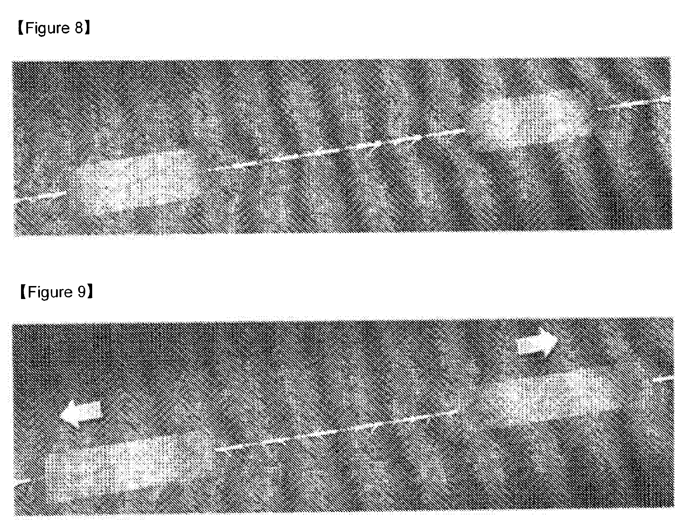

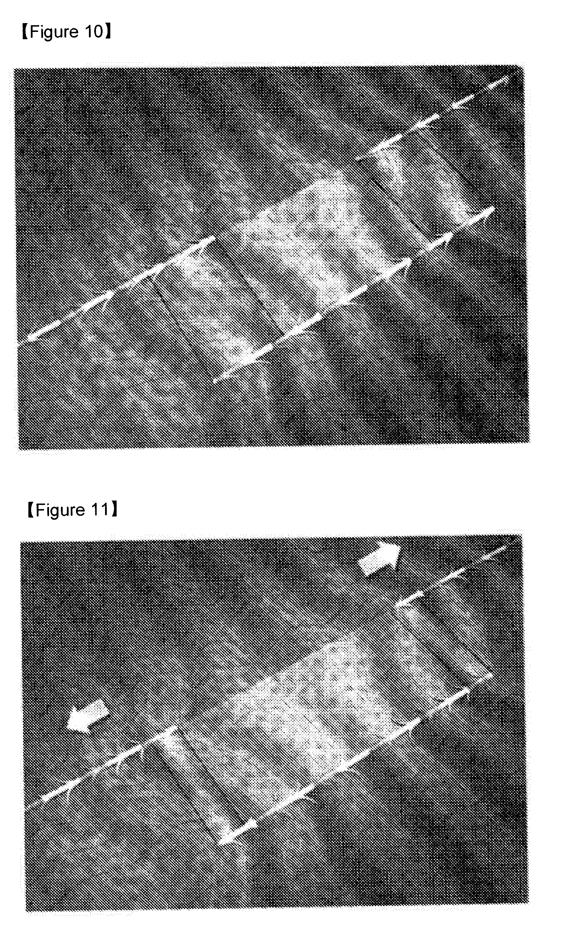

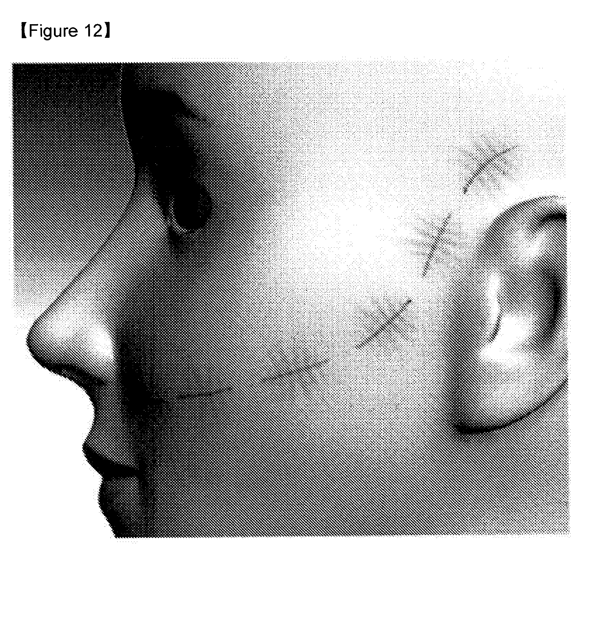

FIG. 4 is a cross-sectional view of an injection needle for plastic surgery prefilled with surgical threads for wrinkle removal according to an embodiment of the present invention, FIG. 5 illustrates various shapes of projections of surgical threads prefilled in injection needles for plastic surgery according to exemplary embodiments of the present invention, FIG. 6 illustrates various cross-sections of surgical threads prefilled in injection needles for plastic surgery according to exemplary embodiments of the present invention, FIG. 7 is a cross-sectional view illustrating a state in which surgical threads for wrinkle removal are prefilled in an injection needle for plastic surgery according to an embodiment of the present invention and a push pin is used to inject the surgical threads into a human body, FIG. 8 shows a state in which surgical threads are injected using an injection needle according to an embodiment of the present invention, FIG. 9 shows surgical threads injected using an injection needle according to an embodiment of the present invention to remove wrinkles, FIG. 10 shows a state in which surgical threads are injected using an injection needle according to another embodiment of the present invention, FIG. 11 shows surgical threads injected using an injection needle according to another embodiment of the present invention to remove wrinkles, and FIG. 12 schematically shows a state in which surgical threads are injected into the skin to remove wrinkles in accordance with the present invention.

The present invention provides an injection needle 100 for plastic surgery that can effectively insert surgical threads 200 into the epidermal layer of the skin. As illustrated in FIG. 4, the injection needle 100 comprises a hollow cylindrical injection portion 102 to be injected into a human body and short surgical threads 202 accommodated in the injection portion 102. The surgical threads 200 have a plurality of directional projections 202.

The injection needle 100 comprises an insertion portion 104 through which a pressure is applied to insert the surgical threads 200 into the human body.

The injection portion 102 has a hollow cylindrical shape, like a general needle, and is formed with a tip at one end thereof. The tip is designed sharp enough to be injected into the human body.

The directional projections 202 of the surgical threads 200 are formed at regular intervals along the axis of each thread. The surgical threads 200 accommodated in the injection portion 102 are 0.8 to 10 cm long.

Each of the surgical threads 200 preferably has a diameter of about 0.25 mm to about 0.80. The surgical threads 200 are accommodated in the injection portion 102 in such a manner that the projections 202 are in close contact with the body of each surgical thread 200. When the injection portion 102 is inserted into the human body, the projections 202 stretch outward from the surgical threads 200 and are fixedly fitted into the soft tissue of the skin.

If the injection portion 102 is much larger in diameter than the surgical threads 200, the preceding surgical thread is not pushed by the succeeding surgical thread, and as a result, the surgical threads overlap each other. Accordingly, it is preferred to appropriately select the diameter of the injection portion 102 depending on the diameter of the surgical threads 200.

Too large a diameter of the injection portion 102 would be undesirable taking into consideration the fact that the injection portion 102 penetrates the skin of the human body. The diameter of the injection portion 102 is preferably limited to 18 to 26 gauges.

The insertion portion 104, where a pressure is applied to the surgical threads 200 accommodated in the injection portion 102, inserts the surgical threads 200 into the human body. As illustrated in FIG. 7, a push pin 300 is inserted into the insertion portion 104 to push the surgical threads 200 into the human body. The push pin 300 is in the form of a pin or an inner needle. The insertion portion 104 is designed to be coupled to a syringe, so that the surgical threads 200 can be inserted into the human body by the pneumatic pressure of the syringe. The syringe may be one that has a tip of a Luer lock structure detachably attached to the injection needle in the insertion portion. Alternatively, the syringe may be a suture syringe in the form of a hair transplanter that has a long cylindrical syringe body, a push needle as pressurization means having a thickness enough to be fitted into the syringe body, a spring, and a plunger. The end of the plunger is designed to have a diameter at least 15 mm wider than the syringe body so as to be suitable to hold it with the ring and little fingers. A plurality of protrusions capable of pulled with the thumb can be formed in the syringe to stepwise control the pulling rate. Due to this construction, the surgical thread can be inserted into the human body.

The projections 202 of the surgical threads 200 may be structurally varied depending on the surgery situation.

FIG. 5 illustrates various shapes of the projections 202 of the surgical threads 200. As illustrated in FIG. 5, the projections 202 may be formed in a row (a) or may be arranged in two rows (b).

In an alternative embodiment, the projections 202 may be arranged in two or more rows (c).

Referring to FIG. 5, the projections 202 may be formed asymmetrically (a) or symmetrically (b) with respect to the axis of the corresponding thread.

In another alternative embodiment, the projections may be arranged helically along the axis of the corresponding thread (c).

The surgical threads 200 may vary in cross section. As illustrated in FIG. 6, the surgical threads 200 may have various shapes, such as triangle, tetragon and lozenge. The polygonal cross-sections of the surgical threads lead to an increase in the adhesion to the epidermal tissue after insertion due to the increased surface area of the surgical threads.

The projections 202 may be formed at corners of the polygonal cross-section of each of the surgical threads 200.

The surgical threads 200 may be made of synthetic resins known in the art. It would be desirable that each of the surgical threads 200 made of a monofilament of polypropylene or nylon harmless to humans.

Each of the surgical threads 200 may be made of a monofilament of a biodegradable polymer selected from polydioxanone (PDS), Monocryl, polycaprolactone (PCL), poly(glycolic acid) (PGA), poly(lactic acid) (PLA) and poly(lactic-co-glycolic acid) (PLGA).

The injection needle of the present invention can insert a plurality of surgical threads at one time to remove wrinkles and is effective in inserting the surgical threads in a row. As shown in FIG. 8, the surgical threads can be inserted at regular intervals in a row.

Like an elastic metal band including non-elastic metal plates and an elastic spring connecting the metal plates, elastic epidermal tissues are located between the short surgical threads whose elasticity is negligible to impart certain elasticity over the entire region of the surgical threads. That is, when the skin, into which the surgical threads have been inserted, is stretched, the epidermal tissues between the surgical threads are stretched, as shown in FIG. 9, leading to an increase in the overall region of the surgical threads.

The use of the short surgical threads prefilled in the injection needle of the present invention enables various treatment operations.

As shown in FIG. 10, beside the interval between the inserted surgical threads, a new short surgical thread may be inserted to more effectively remove wrinkles.

The insertion of the new surgical thread as shown in FIG. 10 can prevent the surgical threads inserted beside the new surgical thread from excessively getting loose, as shown in FIG. 11, to inhibit the formation of new wrinkles and to prevent aging of the skin.

The lateral arrangement of the short surgical threads in the axial direction of the threads enables the removal of wrinkles in a wider area of the skin.

The use of the short surgical threads allows for the removal of wrinkles in a very small area.

The injection needle of the present invention can insert a plurality of surgical threads into the skin at one time, so that the risk of bleeding can be minimized.

After the short surgical threads are inserted into the epidermal tissues of the skin, an external force applied to the inserted surgical threads is rapidly dispersed into the skin because the surgical threads are elastic likewise the soft tissues of the skin. The movement of the surgical threads becomes natural after treatment and the wrinkle removal effect of the surgical threads is continued for a long period of time without being cut.

### [Mode for Invention]

Hereinafter, the present invention will be explained in more detail with reference to the following examples. However, these examples are not intended to limit the present invention.

### Example 1

As shown in FIG. 8, polypropylene surgical threads having a diameter of 0.3 mm and a length of 2 cm were sewn at intervals of 0.5 cm in a row into a non-woven fabric for artificial leather made of polyester microfiber.

### Example 2

As shown in FIG. 10, polypropylene surgical threads having a diameter of 0.3 mm and a length of 2 cm were sewn at intervals of 0.5 cm in a row into a non-woven fabric for artificial leather made of polyester microfiber. Beside the inserted surgical threads, a polypropylene surgical thread was sewn at an interval of 0.5 cm into the non-woven fabric.

### Comparative Example 1

No treatment was performed on a non-woven fabric for artificial leather made of polyester microfiber.

### Comparative Example 2

Long polypropylene surgical threads having a diameter of 0.3 mm were sewn in a row into a non-woven fabric for artificial leather made of polyester microfiber.

### * Tensile strength and elongation measurements

Each of the sewn fabrics of Examples 1 and 2 and Comparative Examples 1 and 2 was cut into a specimen having a width of 2 cm and a length of 8 cm. The tensile strength and elongation at break of the specimen were measured at a tensile rate of 10 cm/min and at a width of 1 cm in accordance with the procedures of JIS L 1096. The results are shown in Table 1.

**TABLE 1**

| | Example 1 | Example 2 | Comparative Example 1 | Comparative Example 2 |
|---|---|---|---|---|
| Tensile strength (kg/cm²) | 89.9 | 90.2 | 89.7 | 90.3 |
| Elongation (%) | 110 | 106 | 101 | 95 |

As can be seen from the results in Table 1, the fabrics sewn with the short surgical threads showed any significant difference in strength but had much higher elongations in comparison with the fabric sewn with the long surgical thread.

These results suggest that wrinkle removal treatment using the short surgical threads can ensure natural movement of the treated skin areas due to the elasticity of the short surgical threads.

Although the present invention has been described herein with reference to the foregoing embodiments and accompanying drawings, the scope of the present invention is not limited to the embodiments and drawings. Therefore, it will be evident to those skilled in the art that various substitutions, modifications and changes are possible, without departing from the spirit of the invention as disclosed in the accompanying claims.

## Claims

1. An injection needle for plastic surgery prefilled with one or more surgical threads for wrinkle removal, designed to have a sharp tip through which the surgical threads are inserted into a human body and a hollow cylindrical injection portion accommodating the surgical threads therein, wherein each of the surgical threads is 0.8 to 10 cm long and has a plurality of projections in different directions that are symmetrically formed with respect to the axis of the thread to gather soft tissues of the skin or to stretch epidermal tissues.

2. The injection needle for plastic surgery according to claim 1, wherein the projections are arranged helically along the axis of each surgical thread.

3. The injection needle for plastic surgery according to claim 1, wherein the injection portion has a diameter of 18 to 26 gauges.

4. The injection needle for plastic surgery according to claim 1, wherein each of the surgical threads has a diameter of 0.25 to 0.80 mm.

5. The injection needle for plastic surgery according to claim 1, wherein each of the surgical threads is made of a monofilament of a biodegradable polymer selected from polydioxanone (PDS), Monocryl, polycaprolactone (PCL), poly(glycolic acid) (PGA), poly(lactic acid) (PLA) and poly(lactic-coglycolic acid) (PLGA).

6. The injection needle for plastic surgery according to claim 1, wherein each of the surgical threads is made of a monofilament of polypropylene or nylon.

7. The injection needle for plastic surgery according to claim 1, wherein each of the surgical threads is a shaped yarn.

8. The injection needle for plastic surgery according to claim 7, wherein the projections are formed at corners of a polygonal cross-section of each surgical thread.
